# EUROPEAN PATENT SPECIFICATION

(11) **EP 1 582 194 B1**
(45) Date of publication and mention of the grant of the patent: **07.07.2010**
(21) Application number: 03775823.2
(22) Date of filing: 14.11.2003
(51) Int. Cl.: A61K 8/04, A61K 8/88, A61Q 1/00

(54) **COSMETIC COMPOSITION**
KOSMETISCHE ZUSAMMENSETZUNG
COMPOSITION COSMETIQUE

(30) Priority: 14.11.2002 JP 2002331177
(43) Date of publication of application: 05.10.2005
(73) Proprietor: Ube Industries, Ltd., Ube-Shi, Yamaguchi 755-8633 (JP)
(72) Inventor: Asano, Yukihiko, Ichihara-shi, Chiba 290-0045 (JP); Nakayama, Kimio, Ichihara-shi, Chiba 290-0045 (JP); Yao, Shigeru, Ichihara-shi, Chiba 290-0045 (JP); Shoji, Tatsuya, Ichihara-shi, Chiba 290-0045 (JP)
(74) Representative: Albrecht, Thomas
(86) International application number: PCT/JP2003/014533
(87) International publication number: WO 2004/043411

(56) References cited:
- EP-A- 0 676 195
- WO-A1-02/067874
- JP-A- 3 284 610
- JP-A- 9 278 627
- JP-A- 62 215 638
- JP-A- 62 221 614
- JP-A- 2001 294 522
- JP-A- 2002 060 312
- JP-A- 2002 080 629
- US-A- 4 831 061
- PATENT ABSTRACTS OF JAPAN vol. 012, no. 128 (C-489), 20 April 1988 (1988-04-20) & JP 62 249914 A (SEKISUI PLASTICS CO LTD), 30 October 1987 (1987-10-30)
- PATENT ABSTRACTS OF JAPAN vol. 012, no. 176 (C-498), 25 May 1988 (1988-05-25) & JP 62 283911 A (SEKISUI PLASTICS CO LTD), 9 December 1987 (1987-12-09)

## Description

### [Field of Invention]

The present invention relates to a cosmetic composition, particularly a cosmetic composition which effectively diminish abnormal light reflection produced on a skin when it is applied to the skin.

### [Background of Invention]

Japanese Patent No. 2653990 claiming a priority of French Patent Application No. 94 03452 describes a cosmetic gel for cleansing mask which contains 12% or more of substantially porous polyamide particles. It is described that the addition of the polyamide particles to the cosmetics can show improved cleansing effect, easy handling and easy removal, and shows reduction of adverse effects to skin surface such as hardening of human skin which occurs when cosmetics containing conventional solid ingredients such as kaolin.

United States Patent 4,831,061 describes a porous polyamide powder having a "gypsum rose" structure wherein polyamide particles have a lamellar or shell-like structure whose lamellae, which grow anarchically are connected to each other, form cavities whose geometric forms are directed toward the center of the particles. It is described that the porous polyamide powder is useful in the use for preparing cosmetics, paints, pharmaceuticals and microcapsules.

WO-02/67874 (US-2004/0071747) discloses cosmetic compositions comprising porous spherical particles such as polyamide particles in a powder base.

### [Disclosure of Invention]

The inventors of the present invention have found that use of porous polyamide particles in the form of spheres having a large surface area and a large oil absorbing capacity as additives in cosmetics show excellent light scattering performance and lipid absorbing effect on a skin surface so as to effectively diminish abnormal light reflection on the skin surface and effectively shield defects on the skin such as wrinkles and pits, when the cosmetics are applied to the skin. The present invention has been derived from the above-mentioned new finding.

The present invention resides in a cosmetic composition comprising a cosmetic base selected from the group consisting of liquid base, paste base and powder base, a perfume ingredient, and spherical porous polyamide-6 particles having a number average particle diameter of 1 to 30 µm, a BET specific surface area of 5 m²/g or larger, a boiled linseed oil absorption of 200 mL/100 g or larger, a crystallinity of 40% or higher (measured by DSC), and a ratio of volume average particle diameter/number average particle diameter of 1.0 to 1.5.

The "boiled linseed oil absorption" defined in the invention is an oil absorption amount determined according to the procedure described in JIS K5101.

The BET surface area of the porous polyamide particles employed in the invention preferably is 8 m²/g or larger. The boiled linseed oil absorption preferably is 240 mL/100 g or larger, more preferably 250 mL or larger. The crystallinity preferably is 45% or higher, more preferably 50% or higher.

### [Best Embodiments performing Invention]

The present invention provides an improvement of cosmetic compositions such as foundations for diminishing abnormal light reflection on skin and making skin surface even and cosmetics comprising dyes, pigments and medical ingredients.

The cosmetic composition of the invention contains porous polyamide-6 particles and is applicable to skin, lips, head skin, eyelashes, eyes, nails and hair. Examples of the cosmetic compositions include those in the forms paste, powder and emulsion which are easily coated on skin.

The cosmetic composition of the invention preferably contains 1 to 60 wt.% of the porous polyamide-6 particles and 3 to 10 wt.% of an oily vehicle.

When the cosmetic composition of the invention is employed as a colored cosmetic composition, the composition preferably contains 1 to 60 wt.% of the porous polyamide-6 particles, 3 to 10 wt.% of an oily vehicle and 1 to 30 wt.% of pigments. The porous polyamide-6 particles are preferably colored. For example, the porous polyamide-6 particles can be colored with an organic dye such as a dye of red, green or yellow.

When the cosmetic composition of the invention is formulated as foundation, the composition preferably contains 1 to 10 wt.% of the porous polyamide-6 particles and 1 to 15 wt.% of an inorganic filler, more preferably 2 to 9 wt.% of the porous polyamide-6 particles and 1 to 15 wt.% of an inorganic filler.

The cosmetic composition of the invention can be formulated as a cosmetic composition for treating skin disease. In this case, the cosmetic composition preferably contains 5 to 20 wt.% of the porous polyamide particles and 0.2 to 15 wt.% of a medically active ingredient, more preferably 5 to 20 wt.% of the porous polyamide particles and 0.1 to 20 wt.% of a medically active ingredient.

When the cosmetic composition of the invention is employed for treating disease, the effect of the medically active ingredient is increased, the treating effect is kept for a long period, and the cosmetic effects are kept for a long period because of increased holding of sweat and secreting fluid. As the medically active ingredients, known medically active ingredients having functions of defatting and wetting skin, decoloring skin, shielding pits of skin, softening skin, treating skin diseases, or imparting perfume can be employed.

The cosmetic composition for foundation is directly coated on skin for pretreatment of make-up to make unevenness on skin smooth and shield defects and pits of skin, and serves as an underlying layer for keeping overcoat cosmetics tightly on the skin.

The cosmetic composition for foundation can be in the form of a creamy composition, an aqueous solution, an emulsion or a gel.

The cosmetic composition for foundation can contain the porous polyamide-6 particles and an inorganic filler, and, if necessary, a fatty acid, an emulsion, a silicone oil, a water-soluble polymer, a pigment and an organic additive.

The fatty acid can be a saturated fatty acid, an unsaturated fatty acid, or a derivative thereof. Examples of the fatty acids include caprylic acid, lauric acid, myristic acid, palmitic acid, stearic acid, and a mixture thereof. Examples of the unsaturated fatty acids include palmitolic acid, myristoleic acid, oleic acid and a mixture thereof, and poly-unsaturated acids such as linoleic acid, linolenic acid and erucic acid. The fatty acid derivative can be a hydroxylated compound or an ester compound.

The emulsion preferably is a water-in-oil emulsion. The oil phase can comprise a silicone oil, a non-silicone oil such as a mineral oil, a vegetable oil, beeswax, fat or wax, or a mixture thereof. The silicone oil can be a volatile silicone oil or a non-volatile silicone oil.

The inorganic filler can be titanium white, silica, talc, kaolin, mica or diatomaceous earth.

The water soluble polymer can be poly(vinyl alcohol), poly(vinyl pyrrolidone), or poly(acrylcarboxylic acid) compound.

The pigment can be a pigment of iron oxide.

The organic additive can be a wetting agent such as amino acid or urea, perfume, dye, or antiseptic.

The oily vehicle can be paraffin, polyethylene wax, liquid paraffin, carnauba wax, vegetable oil, beeswax, silicone oil, or an aliphatic alcohol.

The cosmetic composition of the invention may contain an appropriate amount of an inorganic pigment, a dispersant, antiseptics, or an oxidation inhibitor.

The cosmetic composition can contain a water miscible solvent such as glycerol, ethylene glycol, propylene glycol, dipropylene glycol, 1,3-butylene glycol or sorbitol, a fat or oil such as an aliphatic acid, an aliphatic alcohol, wax (e.g., beeswax), or a derivative thereof, an oily medium such as a vegetable oil (e.g., tsubaki oil), an aqueous medium, or an emulsion medium. Examples of the aqueous media include water and various alcohols such as ethanol. The cosmetic composition may contain an inorganic powder such as talc or clay, an organic powder, or a fibrous powder for diminishing or increasing gloss, removing sweat, or preventing exfoliation, in addition to the porous polyamide particles.

The cosmetic composition can comprise an active compound for treating oily skin. The active compound can be β-lactam compound, ciprofloxacillin, n-floxacillin, tetracycline or its salt, erythromycin or its salt, or an extract from a plant.

The composition of the invention preferably contains 10 to 300 weight parts of a liquid medium per 100 weight parts of the porous polyamide particles. The liquid medium preferably is an oily medium or latex. Examples of the liquid media include volatile or non-volatile silicone oil, liquid paraffin, vegetable oil, wax, glycerol, and ethylene glycol.

The cosmetic composition can be employed for cleansing skin coated with cosmetics.

The porous polyamide particles are spherical particles.

The porous polyamide particles are polyamide 6 particles.

The porous polyamide particles employed in the invention can be prepared basically according to the process described in Japanese Patent Provisional Publication 2002-80629 and selecting appropriate preparation conditions.

The porous polyamide particles have a number average particle size in the range of 1 to 30 µm, more preferably in the range of 2 to 20 µm. Porous polyamide particles having a number average particle size less than the above-mentioned range cannot satisfactorily shield pits on skin and show poor dispersability so that the treated skin would appear uneven. Porous polyamide particles having a number average particle size larger than the above-mentioned range make the treated skin uneven. Moreover, cosmetic compositions containing the larger porous polyamide particles cannot impart satisfactory feeling to skin. A ratio (PDI) of a volume average particle size (Dv) to a number average particle size (Dn) [PDI=Dv/Dn] is in the range of 1.0 to 1.5, preferably in the range of 1.0 to 1.3, for the porous polyamide-6 particles. Porous polyamide particles having PDI 1 or near to 1 show high dispersability, and hence they are preferably incorporated into cosmetic compositions from the viewpoints of preparation and use of the cosmetic composition.

The porous polyamide particles preferably have an average pore size in the range of 0.01 to 0.2 µm, more preferably in the range of 0.02 to 0.1 µm.

The porous polyamide-6 particles have a high crystallinity and hence show a sufficient hardness. Accordingly, the porous polyamide particles are hardly deformed and hardly collapse in the course of preparing and using the cosmetic composition.

The porous polyamide particles can be prepared according to the process described in Japanese Patent Provisional Publication 2002-80629. In more detail, a polyamide solution is mixed with a non-solvent for polyamide and an appropriate amount of water to give temporarily a clear homogeneous solution from which polymer particles precipitate. Polyamide of 0.1 to 30 wt.% and a solvent of 99.8 to 70% can give the polyamide solution (100 wt.%). Preferably, polyamide of 0.2 to 25 wt.% and a solvent of 99.8 to 75 wt.% gives the polyamide solution (100 wt.%).

Examples of the solvents for polyamide include aromatic alcohols and formic acid. The aromatic alcohols can be o-cresol, m-cresol, p-cresol, chlorophenol, phenol, or a mixture thereof.

The non-solvent for polyamide can be partly miscible with (or partly soluble in) an aromatic alcohol and water. Examples of the non-solvents include aliphatic alcohols, aliphatic ketones, and mixtures thereof. It is important for the non-solvent for polyamide to be miscible with water. Examples of the non-solvents include aliphatic alcohols and ketones having 1 to 4 carbon atoms such as methanol, ethanol, propanol, isopropanol, acetone and methyl ethyl ketones. Mixtures of these solvents can be also employed.

In the preparation of the porous polyamide particles, the non-solvent of 98 to 10 wt.% and water of 2 to 90 wt.% are preferably employed. The polyamide solution, non-solvent and water can be mixed in any orders and procedures under such condition that the resulting mixture becomes clear homogeneous solution

For instance, the porous polyamide particles can be prepared by one of the following methods:
(1) a non-solvent and water are added successively to a polyamide solution;
(2) a mixture of a non-solvent and water is added to a polyamide solution; and
(2) water and a non-solvent are added successively to a polyamide solution.

In more detail, the porous polyamide particles can be produced by mixing a polyamide solution, a non-solvent and water to give a homogeneous clear solution temporarily and then precipitating the polyamide particles at a lapse of 0.1 sec. to 120 min. It is important to temporarily form a homogeneous solution for the production of the porous polyamide particles.

The temporary formation of the homogeneous clear solution in the process for the production of the porous polyamide solution can be accelerated by appropriate stirring. The temporary formation of the homogeneous clear solution by the mixing of a polyamide solution, a non-solvent and water, and the precipitation of the polymer particles in the process for the production of the porous polyamide solution can be preferably carried out at a temperature of 5 to 70°C.

The precipitated polyamide particles can be isolated from the solution by a conventional procedure such as decantation, centrifugal separation, or filtration. For instance, methanol is added to a solution containing the precipitated polyamide particles, and then the polyamide particles are isolated by decantation or centrifugal separation. The precipitated polyamide particles can be washed with methanol, acetone, or the like several times and then isolated by decantation or centrifugal separation. The isolated polyamide particles can be dried *in vacuo* or by applying hot air.

### [Examples]

### (Measurement of number average particle diameter and Evaluation of porosity]

The particle conditions, size and porosity of the polyamide particles were evaluated and measured by means of a scanning electron microscope (SEM). The particle diameter of the polyamide particle was measured on the SEM photograph. The particle diameter of particle having no spherical shape was determined by measuring a diameter of a circle corresponding to a projected area.

The number average particle diameter (Dn) was determined from 100 particles.

### (Measurement of specific surface area)

The specific surface area of the polyamide particle was measured by BET three-points measurement.

### (Average pore size)

The average pore size was measured in the range of 0.0034 to 400 µm by means of a mercury porosimeter.

### (Crystallinity)

The crystallinity was determined from a heat of fusion calculated from an endothermic peak area in the range of 130 to 235°C, which was obtained by DSC under such conditions that the particles were heated in a flowing nitrogen gas (flow rate: 40 mL/min.) from room temperature at a temperature elevation rate of 10°C/min.

### (Evaluation and judgement of Cosmetics)

Monitor test: Each cosmetic composition was applied to a panel of fifty healthy adult women, and their impressions were collected according to the following criteria:
AA: Effect of improvement is prominent.
BB: Some effect of improvement is approved.
CC: No effect of improvement is approved.

### [Example 1]

A nylon solution of 10 g of polyamide 6 (molecular weight: 13,000) in 200 g of m-cresol was prepared. The nylon solution (100 g), methanol (500 g) and water (100 g) were mixed for one minute under stirring with a magnetic stirrer to give a homogeneous solution. At a lapse of 2 minutes after the stirring, polymer particles began to precipitate. The mixture was allowed to stand for 24 hours to terminate the precipitation of polymer particles. The precipitated polymer particles were washed with warmed methanol and acetone and then isolated by centrifugal separation.

The isolated polymer particles was a substantially uniform, spherical porous polyamide particles having a number average particle diameter of 7.81 µm, a particle size distribution index (PDI) of 1.08, a specific surface area of 10800 m²/kg and an average pore size of 0.04 µm. Other measured values are set forth in Table 1.

The porous polyamide spherical particles (0.004 g) were placed in ethylene glycol (99.996 g), and the resulting mixture was stirred for one hour by means of a magnetic stirred under application of ultrasonic wave, to give a uniform dispersion. The dispersion was placed in an optical cell (thickness: 10 mm) and measured transmittances (%) of lights of 700 nm, 550 nm and 400 nm, respectively. The transmittances are set forth in Table 2.

### [Example 2]

The procedures of Example 1 were repeated except that a mixture of methanol (71.4 wt.%) and water (14.3 wt.%) was added to the polyamide 6 solution for a period of 30 minutes, to produce porous polyamide 6 spherical particles.

The porous polyamide particles were subjected to the same measurements as described in Example 1.

The isolated polyamide particles had a number average particle diameter of 13.8 µm, a volume average particle diameter of 14.4 µm, a PDI of 1.05, and an average pore size of 0.096 µm, Other measured values are set forth in Tables 1 and 2.

### [Reference Example 3]

A mixture of methanol (55.6 wt.%0 and water (22.2 wt.%) was added under stirring to a 1.0 wt.% polyamide solution of polyamide 6 (molecular weight: 13000) in m-cresol (22.0 wt.%) for a period of 120 sec. After termination of the stirring, the mixture was allowed to stand, to precipitate polymer particles. The precipitated polymer particles were isolated in a centrifugal separator, washed hot alcohol, and dried *in vacuo* for 24 hours, to give porous polyamide 6 dumbbell-shaped particles.

The porous polyamide particles were subjected to the same measurements as described in Example 1.

The isolated polyamide particles had a average particle length of 10.5 µm and an average pore size of 0.135 µm. Other measured values are set forth in Tables 1 and 2.

### [Comparison Example 1]

Polyamide 12 spherical fine particles produced by a conventional process were subjected to the same measurements as described in Example 1.

The produced polyamide particles had a number average particle diameter of 5.9 µm, a volume average particle diameter of 7.8 µm, and a PDI of 1.32. No pores were observed. Other measured values are set forth in Tables 1 and 2.

**Table 1**

| | Polyamide particles appearance porosity | Specific surface area (m²/g) | Crystallinity (%) | Oil absorption (mL/100 g) |
|---|---|---|---|---|
| Ex. 1 | Porous, spherical | 10.8 | 56 | 280 |
| Ex. 2 | Porous, spherical | 15.6 | 57 | 220 |
| Ref. Ex. 3 | Porous, dumbbell-shaped | 10.7 | 58 | 370 |
| Com.1 | Non-Porous, spherical | 1.3 | 30 | 65 |

**Table 2**

| | Polyamide particles appearance, porosity | Light transmittance (%) | | |
|---|---|---|---|---|
| | | 700 nm | 550 nm | 400 nm |
| Ex. 1 | Porous, spherical | 87.82 | 87.90 | 87.60 |
| Ex. 2 | Porous, spherical | 80.25 | 90.16 | 90.03 |
| Ref. Ex. 3 | Porous, dumbbell-shaped | 71.08 | 70.46 | 71.78 |
| Com.1 | Non-Porous, spherical | 93.50 | 93.29 | 92.90 |

### [Example 4]

### (1) Foundation cosmetic composition

A foundation cream was prepared by well mixing the following components A to G and water:
A components: cyclomethycone (22 wt.%), cetyl-methycone (0.2 wt.%)
B components: mica (0.1 wt.%), silica (1 wt.%), titanium oxide (7.5 wt.%), zinc oxide (3 wt.%)
C components: iron oxide pigment (black, 0.17 wt.%), iron oxide pigment (red, 0.52 wt.%), iron oxide pigment (yellow, 1.82 wt.%)
D components: trihydroxystearine (0.3 wt.%), cyclomethycone (1 wt.%)
E component: propylparabene (0.75 wt.%)
F components: porous polyamide particles produced in Example 1 (5.0 wt.%)
G components: glycerol (8 wt.%), poly(vinyl pyrrolidone) (0.5 wt.%), sodium chloride (2.0 wt.%), sodium dehydroacetate (0.3 wt.%), phenoxyethanol (0.25 wt.%), tetrasodium EDTA (0.1 wt.%)
   Water: added to give a cream of 100 wt.%.

### [Comparison Example 2]

### (1) Production of non-porous polyamide particles

Liquid paraffin (130 mL) and sodium stearate (1 g, dispersing aid) were added to laurolactam (50 g). The mixture was heated to 160°C, and sodium (230 mg) and acetylcaprolactam (0.98 mL) were added to the heated mixture. The resulting mixture was kept heated to perform polymerization reaction. The reaction mixture was filtered, washed with a boiled xylene, and then dried *in vacuo.* There were produced polyamide 12 spherical particles having a mean particle size 4.8 µm and a BET specific surface area 1200 m²/kg. There were observed no pores in the particles.

### (2) Foundation cosmetic composition

The procedures for preparing a foundation cosmetic composition of Example 1 were repeated using the above-mentioned polyamide 12 spherical particles.

The prepared foundation cosmetic compositions were subjected to evaluation concerning spreading of the cosmetic composition in the make-up procedure, feeling, and improvement of reduction of wrinkles. The results are set forth in Table 3.

**Table 3**

| | Reduction of wrinkle | Spreading | Feeling |
|---|---|---|---|
| Example 4 | AA | AA | AA |
| Com.Ex. 2 | CC | BB | CC |

### [Example 5] Lip cream

### (1) Preparation of colored powder

The porous polyamide particles (40 g) prepared in Example 1 were placed in an aqueous mixture comprising Red 106 pigment (1 g), benzyl alcohol (5 g), N-methyl-2-pyrrolidone (10 g) and water (84 g) and heated to 50°C for 20 min. The resulting colored particles were collected on a filter, washed, and dried at 40°C for 24 hours. The resulting particles (96 g) were added to a silicone oil solution (SH 1107C, available from Toray Co., Ltd., 300 g) and stirred. The resulting mixture was dried to give a colored powder.

### (2) Preparation of lip cream

(1) Paraffin 15.0 wt.%, (2) candelilla wax 3.0 wt.%, (3) carnauba wax 2.0 wt.%, (4) methylphenylpolysiloxane 40.0 wt.%, (5) liquid paraffin 29.8 wt.%, (6) perfume 0.2 wt.%, (7) oxidation inhibitor: appropriate amount, and (8) the colored powder 10.0 wt.% were treated in the following manner: The materials (1) to (7) were mixed at 80-85°C to give a solution which was then mixed with the colored powder of (8). The resulting mixture was placed in a vessel and cooled to give a solid lip cream.

### [Example 6] Eye shadow

(1) Talc 10.0 wt.%, (2) kaolin 6.0 wt.%, (3) magnesium carbonate 1.0 wt.%, (4) zinc stearate 5.0 wt.%, (5) titanium oxide 2.0 wt.%, (6) mica coated with titanium oxide 20.0 wt.%, (7) the colored powder prepared in Example 5: 50.0 wt.%, (8) sorbitol sesqioleate 1.0 wt.%, (9) liquid paraffin 5.0 wt.%, (10) perfume: small amount, and (11) antiseptics: small amount were treated in the following manner: The materials (1) to (7) were well mixed, and to the resulting mixture were added the materials (8) to (11), to give an eye shadow.

### [Example 7] Cosmetic composition for medical use

The porous polyamide particles of Example 1 were coated 10% of a skin softening agent (isohexadecane), and the coated polyamide particles were employed to give a cream composition.

### [Industrial Utility]

When a cosmetic composition of the invention containing the porous polyamide particles is coated on skin of human being, the polyamide particles reduces abnormal light reflection on the skin due to their high light dispersing effect and efficiently absorbs lipid components oozing from the body due to high oil absorbing capacity. Moreover, since the porous polyamide particles have sufficient hardness, the preparation of a cosmetic composition is easily performed, and the cosmetic composition is resistant to deformation on the skin.

## Claims

1. A cosmetic composition comprising a cosmetic base selected from the group consisting of liquid base, paste base and powder base, a perfume ingredient, and polyamide particles wherein the polyamide particles comprise spherical porous polyamide-6 particles having a number average particle diameter of 1 to 30 µm, a BET specific surface area of 5 m²/g or larger, a boiled linseed oil absorption of 200 mL/100 g or larger, a crystallinity as measured by DSC of 40% or higher, and a ratio of volume average particle diameter/number average particle diameter of 1.0 to 1.5.

2. The cosmetic composition of claim 1, wherein the BET surface area of the porous polyamide-6 particles is 8 m²/g or larger.

3. The cosmetic composition of claim 1, wherein the boiled linseed oil absorption of the porous polyamide-6 particles is 240 mL/100 g or larger.

## Patentansprüche

1. Kosmetische Zusammensetzung, umfassend eine kosmetische Grundlage, ausgewählt aus der Gruppe, bestehend aus einer flüssigen Grundlage, einer Pastengrundlage und einer Pulvergrundlage, ein Parfumingrediens und Polyamidpartikel, wobei die Polyamidpartikel sphärische, poröse Polyamid-6-Partikel umfassen, die einen zahlenmittleren Partikeldurchmesser von 1 bis 30 µm, eine spezifische BET-Oberfläche von 5 m²/g oder größer, eine Absorption von gekochtem Leinsamenöl von 300 ml/100 g oder größer, eine Kristallinität, wie sie durch DSC gemessen wird, von 40% oder höher und ein Verhältnis von volumenmittlerem Partikeldurchmesser/zahlenmittlerem Partikeldurchmesser von 1,0 bis 1,5 haben.

2. Kosmetische Zusammensetzung nach Anspruch 1, wobei die BET-Oberfläche der porösen Polyamid-6-Partikel 8 m²/g oder größer ist.

3. Kosmetische Zusammensetzung nach Anspruch 1, wobei die Absorption von gekochtem Leinsamenöl durch die porösen Polyamid-6-Partikel 240 ml/100 g oder größer ist.

## Revendications

1. Composition cosmétique comprenant une base cosmétique choisie parmi le groupe consistant en une base liquide, une base pâteuse, un ingrédient de parfum et des particules de polyamide, dans laquelle les particules de polyamide comprennent des particules de polyamide 6 sphériques poreuses ayant un diamètre moyen en nombre de 1 à 30µm, une surface spécifique selon la méthode BET de 5m²/g ou plus, une absorption d'huile de lin bouillie de 200ml/100g ou plus, une cristallinité de 40% ou plus, qui est mesurée par DSC, et un ratio du diamètre moyen en volume par rapport au diamètre moyen en nombre de 1,0 à 1,5.

2. Composition cosmétique selon la revendication 1, dans laquelle la surface spécifique selon la méthode BET des particules poreuses de polyamide 6 est 8m²/g ou plus.

3. Composition cosmétique selon la revendication 1, dans laquelle l'absorption d'huile de lin bouillie des particules poreuses de polyamide 6 est 240ml/100g ou plus.
